# FASCICULE DE BREVET EUROPEEN

(11) **EP 1 299 037 B1**
(45) Date de publication et mention de la délivrance du brevet: **27.04.2005**
(21) Numéro de dépôt: 01955411.2
(22) Date de dépôt: 12.07.2001
(51) Int. Cl.: A61B 17/16, A61B 17/22, A61B 17/32

(54) **OUTIL DE CURETAGE**
KÜRETTE
CURETTAGE INSTRUMENT

(30) Priorité: 12.07.2000 FR 0009095
(43) Date de publication de la demande: 09.04.2003
(73) Titulaire: Spine Next, La Cité Mondiale, 33000 Bordeaux (FR)
(72) Inventeur: PASQUET, Denis, F-33600 Pessac (FR); LE COUEDIC, Régis, F-33000 Bordeaux (FR)
(74) Mandataire: Dronne, Guy
(86) Numéro de dépôt international: PCT/FR2001/002259
(87) Numéro de publication internationale: WO 2002/003870

(56) Documents cités:
- WO-A-90/02524
- WO-A-97/38635
- WO-A-99/63891
- DE-A- 2 630 400
- US-A- 5 489 291
- US-A- 5 836 958
- US-A- 6 030 401

## Description

La présente invention concerne un outil de curetage de l'espace intervertébral et plus particulièrement un outil destiné à aviver les plateaux vertébraux.

Le domaine d'application de l'invention est notamment la chirurgie du disque intervertébral pour laquelle il est nécessaire, dans certain cas, de procéder à l'extraction complète du disque intervertébral endommagé afin de le remplacer par un élément prothétique.

Le traitement des disques dégénératifs, notamment, consiste pour certaines indications thérapeutiques à remplacer le disque intervertébral par une prothèse de disque ou par des greffons osseux immobilisés dans une cage intersomatique. Ces interventions sont effectuées lorsque le disque intervertébral ne peut plus assurer sa fonction. Le disque intervertébral doit résister à la transmission des forces de pesanteur en maintenant un espace intervertébral déterminé tout en permettant des mouvements amples et multidirectionnels intervertébraux.

L'acte chirurgical proprement dit nécessite, après dissection et exposition du disque intervertébral endommagé, d'exciser au bistouri le disque et de le retirer en totalité. Ces opérations sont facilitées en écartant les corps vertébraux adjacents l'un par rapport à l'autre. On notera que pour procéder au retrait du disque intervertébral endommagé, on peut éventuellement utiliser l'outil décrit dans le document WO 97/38635, prévu à cet effet. Cet outil peut aussi être utilisé pour décortiquer les vertèbres. Le préambule de la revendication 1 est fondé sur ce document.

Lorsque le traitement nécessite l'insertion d'un greffon entre les vertèbres, les plateaux vertébraux doivent être avivés de manière à ce qu'ils saignent afin de permettre la prise du greffon. Cette opération est. généralement réalisée au moyen d'une curette avec laquelle les plateaux vertébraux sont grattés. Cependant, cet outil produit des rainures sur la paroi des plateaux vertébraux et ne permet pas un avivement uniforme. Par ailleurs, les plateaux vertébraux présentant une forme concave, il est mal aisé de les gratter pour obtenir une surface uniforme.

Pour effectuer une telle opération de curetage, on connaît également l'outil décrit dans le document WO 99/63891, comprenant un manche et une tige, à l'extrémité de laquelle sont montés deux éléments abrasifs de forme circulaire, entraînés en rotation autour d'un axe perpendiculaire à celui dudit manche et de ladite tige, par un moteur associé à un système d'engrenages.

Un objet de la présente invention est de proposer un outil de curetage permettant d'aviver les plateaux vertébraux dans l'espace intervertébral en respectant la forme concave des plateaux, et plus généralement la forme biconcave de l'espace intervertébral.

Pour atteindre ce but, conformément à l'invention, l'outil de curetage destiné à gratter l'espace intervertébral et notamment les plateaux vertébraux, comporte une première partie formant manche et une seconde partie active, ladite seconde partie active, de forme allongée selon un axe principal, présentant une première extrémité reliée à ladite première partie et une seconde extrémité libre, et comprenant au moins une portion longitudinale formant lame tranchante, ladite portion longitudinale s'étendant de ladite première extrémité de ladite seconde partie vers ladite seconde extrémité libre de ladite seconde partie et présentant une partie médiane, la distance qui sépare ladite partie médiane de ladite portion longitudinale dudit axe principal étant supérieure à la distance qui sépare chacune des extrémités de ladite portion longitudinale dudit axe principal, par quoi l'entraînement en rotation de ladite seconde partie contre la surface dudit plateau intervertébral induit le creusement d'une cavité dont la profondeur maximale correspond à ladite distance séparant ladite partie médiane dudit axe principal.

Ainsi, une caractéristique de l'outil de curetage réside dans la forme de ladite portion longitudinale formant lame tranchante qui s'étend de ladite première extrémité à ladite seconde extrémité libre de ladite seconde partie et décrit une courbe dont la distance qui la sépare de l'axe principal présente un maximum au niveau de sa partie médiane. De la sorte, l'entraînement en rotation de ladite seconde partie active autour de son axe principal, induit la rotation de la portion longitudinale formant lame tranchante et permet de trancher la surface tangentielle sur laquelle est appliquée ladite seconde partie, perpendiculairement à son axe principal. On comprend que la surface tranchée, lorsque les positions relatives de ladite surface et dudit axe principal sont fixes, dépend des rayons de courbure de la trajectoire de ladite lame tranchante et de ladite surface tangentielle. Cependant, le rayon de courbure de ladite trajectoire est tel que la surface tranchée est relativement plane par rapport aux surfaces obtenues au moyen des outils de l'art antérieur. En outre, ladite lame tranchante présentant un maximum, la ligne de coupe épouse la forme concave des plateaux vertébraux.

La seconde partie active présentant une forme allongée est un solide de révolution défini par la rotation autour dudit axe principal d'une génératrice courbe comportant un maximum de distance par rapport audit axe principal.

De la sorte la géométrie de la seconde partie active est conforme à la géométrie de l'espace intervertébral biconcave, quelle que soit la position angulaire de ladite seconde partie puisque qu'elle présente une symétrie cylindrique.

Selon une particularité avantageuse, la génératrice présente une variation continue de son rayon de courbure, afin d'obtenir un état de surface des plateaux vertébraux relativement uniforme.

Afin de couvrir l'ensemble de l'espace intervertébral, sensiblement demi-circulaire et limité dans sa partie inférieure et dans sa partie supérieure par deux plateaux vertébraux sensiblement parallèle, la seconde partie active de l'outil doit être actionnée en rotation et déplacée latéralement.

Préférentiellement, la ligne moyenne de ladite portion longitudinale formant lame tranchante, orientée selon le sens de déplacement de ladite portion et contenue dans un plan perpendiculaire audit axe principal, forme un angle avec une demi-droite radiale, orientée dudit axe principal vers ladite portion longitudinale et contenue dans ledit plan perpendiculaire, inférieur à 90°.

Ainsi, selon cette particularité, l'angle de ladite portion formant lame tranchante avec la surface du plateau intervertébral tangentielle à la trajectoire de ladite portion, est il inférieur à 90° ce qui permet d'obtenir, lorsque l'outil est actionné en rotation, un effet tranchant optimal.

Selon un mode particulier de réalisation, ladite portion longitudinale formant lame tranchante est formée par au moins un bord saillant d'un évidement oblong ménagé dans la paroi de ladite seconde partie dudit outil.

Ainsi, le bord saillant de l'évidement oblong ménagé dans la seconde partie active forme une arrête qui peut être usinée de façon à obtenir une lame tranchante.

Avantageusement, ladite seconde partie présente un alésage axial selon l'axe principal et le fond dudit évidement débouche dans ledit alésage. Cette particularité permet d'évacuer les copeaux issus du tranchage de la surface des plateaux vertébraux puisque, lorsque le copeau est réalisé, il traverse le fond de l'évidement et est évacué à l'intérieur de l'alésage. Lorsque la totalité de l'espace alésé est occupée par des copeaux lors de utilisation de l'outil, il est nécessaire de retirer ce dernier de l'espace intervertébral pour dégager les copeaux.

, Selon un autre mode particulier de réalisation ledit évidement oblong est ménagé dans ladite seconde partie selon un plan médian ne contenant pas ledit axe principal de façon à ménager ledit bord saillant. De la sorte, la lame tranchante est formée aisément sans usinage particulier de l'arrête de l'évidement oblong. En effet, en ménageant l'évidement oblong selon un plan médian relativement incliné par rapport à l'axe radial on obtient une arête suffisamment vive pour former la lame tranchante.

Selon un mode particulier de mise en oeuvre de l'invention, ledit évidement oblong est ménagé autour de ladite seconde partie de façon à former une hélice selon l'axe principal. Ainsi, lorsque ladite seconde partie active est entraînée en rotation, la ligne de coupe de la portion longitudinale formant lame tranchante est oblique par rapport à la surface du plateau vertébral à entailler ce qui permet une meilleure coupe.

Selon un autre mode particulier de mise en oeuvre de l'invention, ladite seconde partie présente une pluralité de portions longitudinales, sensiblement parallèles entre elles, formant lames tranchantes. De la sorte, la seconde partie active de l'outil, insérée entre les deux plateaux vertébraux, est apte à aviver simultanément, le plateau supérieur et le plateau inférieur, dans la mesure où les dimensions de ladite seconde partie active correspondent aux dimensions intervertébrales.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique partielle en élévation d'un outil de curetage conforme à l'invention,
- la Figure 2, est la représentation graphique d'une portion de courbe dans un repère (O,x,y), apte à définir un solide de révolution,
- la Figure 3 est une section droite de la seconde partie active, selon l'invention, réalisée au droit de ladite partie médiane de la portion longitudinale, telle que représentée sur la Figure 1, et,
- la Figure 4 est une vue schématique illustrant l'utilisation de l'outil de curetage dans l'espace intervertébral.

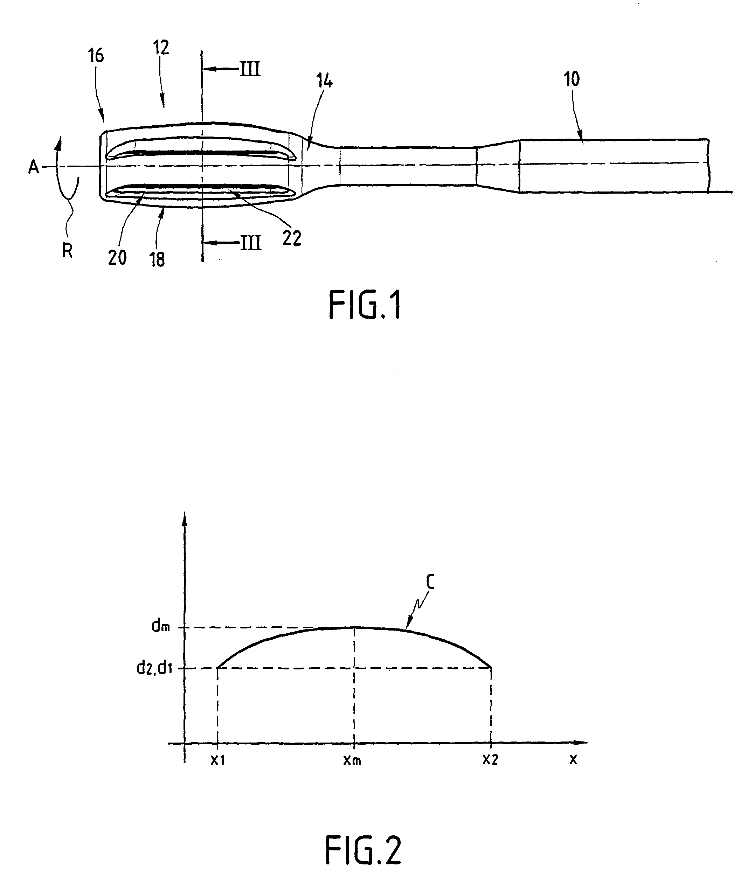

En se référant tout d'abord aux Figures 1 et 2 on décrira l'outil de curetage de façon générale.

Sur la Figure 1 l'outil qui présente une symétrie cylindrique est représenté en élévation et comporte une première partie 10 formant manche, prolongée coaxialement par une seconde partie active 12 ; l'axe commun aux deux parties est dénommé axe principal A. La seconde partie active 12 présente une première extrémité 14 reliée à la partie 10 formant manche et une seconde extrémité libre 16. La seconde partie active 12 comprend à sa périphérie une portion longitudinale 18 formant lame tranchante s'étendant de sa première extrémité 14 à sa deuxième extrémité libre 16.

La portion longitudinale 18 présente une forme concave dont la concavité est dirigée vers l'axe principal A. En se référant à la Figure 2 on décrira de façon générale la forme de la seconde partie active 12 et de façon particulière la forme de la portion longitudinale 18.

Sur la Figure 2 on a représenté une fraction de courbe C présentant un maximum d'abscisse xm et d'ordonnée dm dans sa partie médiane, et deux extrémités d'abscisse x1 et x2 dont les ordonnées d1 et d2 sont sensiblement identiques. L'axe des abscisses est assimilé à l'axe principal A et la seconde partie active 12 est définie par un solide de révolution obtenu par la rotation de la fraction de courbe formant génératrice autour de l'axe des abscisses.

La fraction de courbe C présente une variation continue de son rayon de courbure, qui est maximum dans sa partie médiane et diminue progressivement vers les extrémités d'abscisses x1 et x2.

Selon un mode particulier de réalisation, la courbe C est symétrique par rapport à un axe coupant perpendiculairement l'axe des abscisses en xm.

Comme illustré sur la Figure 1, on ménage au moins un évidement oblong 20 dans la seconde partie active 12, s'étendant de la première extrémité 14 vers la seconde extrémité libre 16. Ainsi, l'évidement réalisé défini deux arêtes opposées 18, 22, dont une,18, est susceptible d'être transformée en lame tranchante.

Compte tenu de la forme particulière de la seconde partie active 12 définie par ledit solide de révolution, les arêtes 18 et 12 présentent une forme qui se confond avec celle de la portion de courbe C. Ainsi, l'arête tranchante formant lame tranchante 18 présente dans sa partie médiane un maximum correspondant au maximum de la courbe C.

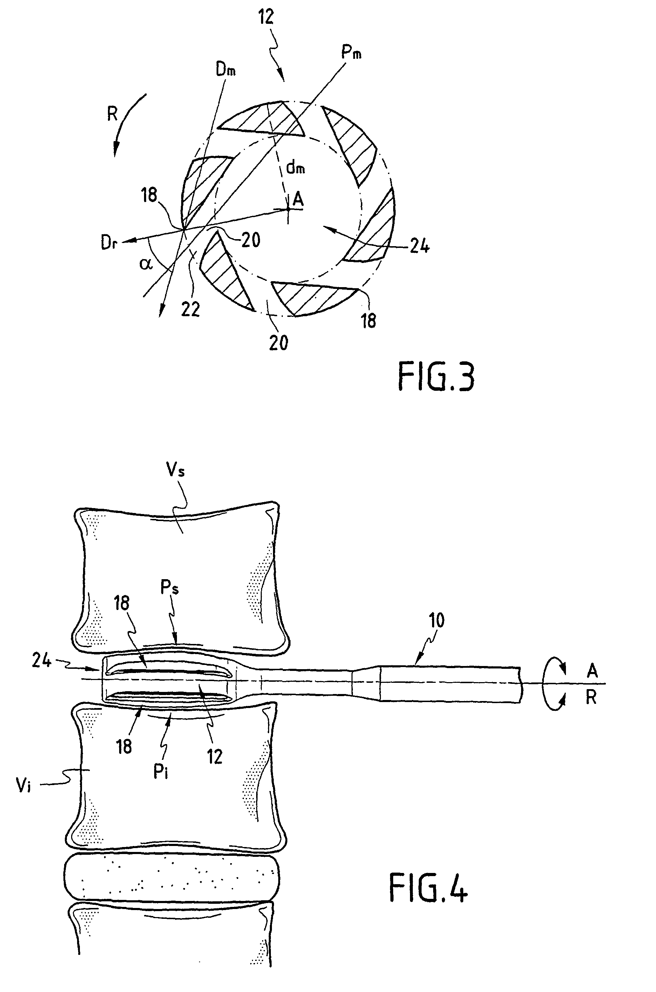

En se référera maintenant à la Figure 3, correspondant à une section droite de la seconde partie active 12 dans sa partie médiane pour décrire un mode particulier de réalisation de l'invention dans lequel ladite seconde partie 12 présente six portions longitudinales 18, sensiblement parallèles entre elles, formant lames tranchantes.

Telle que représentée, on retrouve la seconde partie active 12 qui présente un alésage central 24 dans lequel débouchent six évidements oblongs répartis à la périphérie de ladite seconde partie active 12. On retrouve également la portion longitudinale 18 formant lame tranchante et le bord opposé 22 de l'évidement oblong 20.

Les évidements oblongs 20, ménagés longitudinalement dans la paroi de la seconde partie active selon un plan médian Pm représenté en coupe sur la Figure 3. Ce plan médian Pm ne contient pas l'axe A et il est suffisamment oblique par rapport à la paroi de la seconde partie active 12 pour constituer un bord saillant formant lame tranchante. Ainsi, la ligne moyenne orientée Dm de la portion formant lame tranchante et la demi-droite radiale orientée Dr forment un angle α inférieur à 90°.

On comprend que la rotation de la seconde partie active 12 de l'outil dans le sens R, trigonométrique, induit le déplacement des six bords saillants 18 formant lame tranchante. Ainsi, deux surfaces tangentes en deux points diamétralement opposés de la seconde partie active 12 sont elles entaillées simultanément par les portions 18 formant lames tranchantes. En outre, les six bords saillants permettent de réaliser des entailles avec un nombre limité de rotations de l'outil.

On se référera maintenant à la Figure 4 pour décrire les conditions d'utilisation de l'outil selon l'invention. On retrouve sur la Figure 4 l'outil de curetage tel que décrit ci-dessus en référence à la Figure 1, intercalé entre deux vertèbres adjacentes Vs et Vi.

Après que le disque intervertébral défectueux a été partiellement ou complètement retiré, la seconde partie active 12 de l'outil de curetage est insérée entre les plateaux vertébraux Ps et Pi des deux vertèbres Vs et Vi, lesdits plateaux vertébraux définissant un espace intervertébral biconcave. La forme de la seconde partie active 12 de l'outil de curetage correspond à la forme biconcave de l'espace intervertébral de sorte que la paroi du plateau vertébral supérieur Ps est tangente à la partie active 12 et que la paroi du plateau vertébral inférieur Pi est tangente à la partie active 12 en un point diamétralement opposé.

Selon cette configuration, l'entraînement en rotation de la première partie formant manche 10 selon le sens trigonométrique R, tout en maintenant fixe l'axe A de l'outil, provoque le déplacement des portions longitudinales 18 formant lames tranchantes contre les plateaux vertébraux Ps et Pi. De la sorte, la ligne de coupe de ladite lame tranchante entaille les plateaux Ps et Pi et les copeaux générés par les portions longitudinales formant lames tranchantes pénètrent dans l'alésage 24.

On comprend que les lames tranchantes forment des entailles longitudinales dans la paroi des plateaux vertébraux Ps et Pi, dont la largeur dépend du rayon de la seconde partie active 12, qui est déterminé par l'espace intervertébral disponible.

Afin d'aviver la totalité de la paroi des plateaux vertébraux, l'outil de curetage doit être déplacé latéralement tout en étant actionné en rotation. L'avivement des plateaux vertébraux doit conduire à leur saignement de façon que le greffon osseux qui sera inséré entre les deux plateaux puisse prendre.

Selon un mode de réalisation particulier, les évidements oblongs sont ménagés autour de la seconde partie active de façon à former une hélice selon l'axe principal A. Cette disposition peut dans certains cas améliorer le tranchage de la paroi des plateaux vertébraux car la portion formant lame tranchante est oblique par rapport à la paroi et la ligne de coupe a tendance à glisser dans la partie entaillée.

Par ailleurs, l'outil de curetage conforme à l'invention peut être réalisé en acier inoxydable ou dans tout autre matériau solide.

## Revendications

1. Outil de curetage destiné à gratter l'espace intervertébral et notamment les plateaux vertébraux, comportant une première partie (10) formant manche et une seconde partie active (12),
**caractérisé en ce que** ladite seconde partie active (12), forme un solide de révolution défini par la rotation d'une génératrice courbe (C) autour d'un axe principal (A), ladite génératrice comportant un maximum (dm) de distance par rapport audit axe principal (A), **en ce que** ladite seconde partie active (12), allongée selon ledit axe principal (A) entre une première extrémité (14) reliée à ladite première partie (10) et une seconde extrémité libre (16), comprend à sa périphérie au moins une portion longitudinale (18) formant lame tranchante, ladite portion longitudinale (18) s'étendant de ladite première extrémité (14) de ladite seconde partie (12) vers ladite seconde extrémité libre (16) de ladite seconde partie (12) et présentant une partie médiane,
et **en ce que** la distance (dm) qui sépare ladite partie médiane de ladite portion longitudinale (18) dudit axe principal (A) est supérieure à la distance (d1, d2) qui sépare chacune des extrémités de ladite portion longitudinale (18) dudit axe principal (A), de sorte que ladite portion longitudinale (18) formant lame tranchante a une forme courbe, par quoi l'entraînement en rotation de ladite seconde partie (12) contre la surface dudit plateau intervertébral induit le creusement d'une cavité dont la profondeur maximale correspond à ladite distance (dm) séparant ladite partie médiane dudit axe principal (A).

2. Outil de curetage selon la revendication 1, **caractérisé en ce que** ladite génératrice (C) présente une variation continue de son rayon de courbure.

3. Outil de curetage selon la revendication 1 ou 2, **caractérisé en ce que** la ligne moyenne (Dm) de ladite portion longitudinale (18) formant lame tranchante, orientée selon le sens de déplacement de ladite portion et contenue dans un plan perpendiculaire audit axe principal (A), forme un angle (α) avec une demi-droite radiale (Dr), orientée dudit axe principal (A) vers ladite portion longitudinale (18) et contenue dans ledit plan perpendiculaire, inférieur à 90°.

4. Outil de curetage selon l'une quelconque des revendications 1 à 3, **caractérisé en ce que** ladite portion longitudinale (18) formant lame tranchante est formée par au moins un bord saillant (18) d'un évidement oblong (20) ménagé dans la paroi de ladite seconde partie (12) dudit outil.

5. Outil de curetage selon la revendication 4, **caractérisé en ce que** ladite seconde partie présente (12) un alésage axial (24) selon l'axe principal (A) et **en ce que** le fond dudit évidement (20) débouche dans ledit alésage (24).

6. Outil de curetage selon la revendication 4 ou 5, **caractérisé en ce que** ledit évidement oblong (20) est ménagé dans ladite seconde partie (12) selon un plan médian (Pm) ne contenant pas ledit axe principal (A) de façon à ménager ledit bord saillant (18).

7. Outil de curetage selon l'une quelconque des revendications 4 à 6, **caractérisé en ce que** ledit évidement oblong (20) est ménagé autour de ladite seconde partie (12) de façon à former une hélice selon l'axe principal (A).

8. Outil de curetage selon l'une quelconque des revendications 1 à 7, **caractérisé en ce que** ladite seconde partie (12) présente une pluralité de portions longitudinales (18), sensiblement parallèles entre elles, formant des lames tranchantes décalées angulairement par rapport audit axe principal (A).

9. Outil de curetage selon l'une quelconque des revendications 1 à 7, **caractérisé en ce que** ladite seconde partie (12) présente six portions longitudinales (18), sensiblement parallèles entre elles, formant lames tranchantes.

10. Outil de curetage selon l'une quelconque des revendications 1 à 9, **caractérisé en ce que** ledit axe principal (A) est confondu avec celui dudit manche.

## Patentansprüche

1. Kürette, die dazu dient, den Intervertebralraum aus- und insbesondere die Wirbeideckplatten abzuschaben, die ein erstes Teil (10), das den Griff bildet, und ein zweites, aktives Teil (12) aufweist,
**dadurch gekennzeichnet, dass** das zweite, aktive Teil (12) einen Rotationskörper bildet, der durch die Rotation einer generierenden Kurve (C) um eine Hauptachse (A) definiert ist, wobei die Generierende ein Maximum (dm) an Abstand in Bezug auf die Hauptachse (A) aufweist, derart, dass das zweite, aktive Teil (12), das entlang der Hauptachse (A) zwischen einem ersten Ende (14), das mit dem ersten Teil (10) verbunden ist, und einem zweiten freien Ende (16) verlängert ist, an seiner Peripherie wenigstens einen Längsabschnitt (18) aufweist, der die Schneidklinge bildet, wobei der Längsabschnitt (18) sich von dem ersten Ende (14) des zweiten Teils (12) in Richtung auf das zweite freie Ende (16) des zweiten Teils (12) erstreckt und ein Mittelteil aufweist,
**dadurch gekennzeichnet, dass** der Abstand (dm), der das Mittelteil des Längsabschnitts (18) von der Hauptachse (A) trennt, größer als der Abstand (d1, d2) ist, der jedes Ende des Längsabschnitts (18) von der Hauptachse (A) trennt, derart, dass der Längsabschnitt (18), der die Schneidklinge bildet, bogenförmig ist, wodurch das Versetzen in Drehung des zweiten Teils (12) gegen die Oberfläche der Wirbeldeckplatte die Aushöhlung eines Hohlraums bewirkt, dessen maximale Tiefe dem Abstand (dm) entspricht, der das Mittelteil von der Hauptachse (A) trennt.

2. Kürette nach Anspruch 1, **dadurch gekennzeichnet, dass** der Biegeradius der Generierenden (C) eine kontinuierliche Veränderung aufweist.

3. Kürette nach Anspruch 1 oder 2, **dadurch gekennzeichnet, dass** die Mittellinie (Dm) des Längsabschnitts (18), der die Schneidklinge bildet, in Richtung der Verschiebung des Abschnitts ausgerichtet und sich in einer zu der Hauptachse (A) senkrechten Ebene befindet, einen Winkel (α) kleiner als 90° mit der radialen Halbgeraden (Dr) bildet, die zur Hauptachse (A) in Richtung des Längsabschnitts (18) ausgerichtet ist und sich in der senkrechten Ebene befindet.

4. Kürette nach einem der Ansprüche 1 bis 3, **dadurch gekennzeichnet, dass** der Längsabschnitt (18), der die Schneidklinge bildet, durch wenigstens einen scharfen Rand (18) einer länglichen Aussparung (20) gebildet ist, die in der Wand des zweiten Teils (12) der Kürette angeordnet ist.

5. Kürette nach Anspruch 4, **dadurch gekennzeichnet, dass** das zweite Teil (12) eine axiale Bohrung (24) in Richtung der Hauptachse (A) aufweist und dass der Boden der Aussparung (20) in die Bohrung (24) mündet.

6. Kürette nach Anspruch 4 oder 5, **dadurch gekennzeichnet, dass** die längliche Aussparung (20) in dem zweiten Teil (12) entlang einer Mittelebene (Pm) angeordnet ist, die die Hauptachse (A) nicht einschließt, um den scharfen Rand (18) anzuordnen.

7. Kürette nach einem der Ansprüche 4 bis 6, **dadurch gekennzeichnet, dass** die längliche Aussparung (20) um den zweiten Teil (12) derart angeordnet ist, dass eine Spirale in Richtung Hauptachse (A) gebildet wird.

8. Kürette nach einem der Ansprüche 1 bis 7, **dadurch gekennzeichnet, dass** das zweite Teil (12) eine Vielzahl von länglichen, im Wesentlichen parallel zueinander verlaufenden Abschnitten (18) aufweist, die die Schneidklingen bilden, die in einem Winkel zur Hauptachse (A) versetzt sind.

9. Kürette nach einem der Ansprüche 1 bis 7, **dadurch gekennzeichnet, dass** das zweite Teil (12) sechs längliche, im Wesentlichen parallel zueinander verlaufende Abschnitte (18) aufweist, die Schneidklingen bilden.

10. Kürette nach einem der Ansprüche 1 bis 9, **dadurch gekennzeichnet, dass** die Hauptachse (A) mit der des Griffs zusammenfällt.

## Claims

1. A curettage tool designed to scrape an intervertebral space and in particular to scrape vertebral plates, the tool comprising a handle-forming first portion (10) and an active second portion (12),
the tool being **characterised in that** said active second portion (12) forms a solid of revolution defined by rotating a generator line curve (C) about a main axis (A), said generator line including a distance maximum (dm) relative to said main axis (A), **in that** said second active portion (12) is of elongate shape along said main axis (A) between a first end (14) connected to said first portion (10) and a second end (16) that is free, and comprises on its periphery at least one longitudinal portion (18) forming a scraper blade, said longitudinal portion (18) extending from said first end (14) of said second portion (12) to said free second end (16) of said second portion (12) and presenting a middle portion,
and **in that** the distance (dm) between said middle portion of said longitudinal portion (18) and said main axis (A) is greater than the distance (d1, d2) between each of the ends of said longitudinal portion (18) and said main axis (A), such that said longitudinal portion (18) forming a scraper blade is shaped as a curve, whereby rotating said second portion (12) against the surface of said intervertebral plate causes a cavity to be hollowed out having a maximum depth that corresponds to said distance (dm) between said middle portion and said main axis (A).

2. A curettage tool according to claim 1, **characterised in that** said generator line (C) presents a radius of curvature that varies continuously.

3. A curettage tool according to claim 1 or 2, **characterised in that** the mean line (Dm) of said scraper-forming longitudinal portion (18), directed in the travel direction of said portion and contained in a plane perpendicular to said main axis (A), forms an angle (α) with a radial half line (Dr), directed away from said main axis (A) towards said longitudinal portion (18) and contained in said perpendicular plane, which angle is less than 90°.

4. A curettage tool according to any one of claims 1 to 3, **characterised in that** said scraper-forming longitudinal portion (18) is formed by at least one projecting edge (18) of an oblong recess (20) formed in the wall of said second portion (12) of said tool.

5. A curettage tool according to claim 4, **characterised in that** said second portion (12) presents an axial bore (24) along the main axis (A), and **in that** the bottom of said recess (20) opens out to said bore (24).

6. A curettage tool according to claim 4 or 5, **characterised in that** said oblong recess (20) is formed in said second portion (12) on a mid plane (Pm) that does not contain said main axis (A) so as to form said projecting edge (18).

7. A curettage tool according to any one of claims 4 to 6, **characterised in that** said oblong recess (20) is formed around said second portion (12) so as to form a helix along the main axis (A).

8. A curettage tool according to any one of claims 1 to 7, **characterised in that** said second portion (12) presents a plurality of scraper-forming longitudinal portions (18) that are substantially parallel to each other and angularly offset with respect to said main axis (A).

9. A curettage tool according to any one of claims 1 to 7, **characterised in that** said second portion (12) presents six scraper-forming longitudinal portions (18) that are substantially parallel to each other.

10. A curettage tool according to any one of claims 1 to 9, **characterised in that** said main axis (A) coincides with the axis of said scraper.
